# FASCICULE DE BREVET EUROPEEN

(11) **EP 1 147 109 B1**
(45) Date de publication et mention de la délivrance du brevet: **02.04.2003**
(21) Numéro de dépôt: 00900581.0
(22) Date de dépôt: 14.01.2000
(51) Int. Cl.: C07D 487/04, A61K 7/13

(54) **NOUVELLES BASES D'OXYDATION CATIONIQUES, LEUR UTILISATION POUR LA TEINTURE D'OXYDATION DES FIBRES KERATINIQUES, COMPOSITIONS TINCTORIALES ET PROCEDES DE TEINTURE**
KATIONISCHE OXIDATIONSBASEN, DEREN VERWENDUNG ALS OXIDATIVE FÄRBEMITTEL VON KERATINFASERN, FÄRBEMISCHUNGEN UND VERFAHREN ZUM FÄRBEN
NOVEL CATIONIC OXIDATION BASES, THEIR USE FOR OXIDATIVE DYEING OF KERATIN FIBRES, DYEING COMPOSITIONS AND DYEING METHODS

(30) Priorité: 19.01.1999 FR 9900503
(43) Date de publication de la demande: 24.10.2001
(73) Titulaire: L'OREAL, 75008 Paris (FR)
(72) Inventeur: TERRANOVA, Eric, F-92270 Bois Colombes (FR); FADLI, Aziz, F-77550 Chelles (FR); LAGRANGE, Alain, F-77700 Coupvray (FR)
(74) Mandataire: Fevrier, Murielle
(86) Numéro de dépôt international: FR0000073
(87) Numéro de publication internationale: WO00043396

(56) Documents cités:
- EP-A- 0 926 149
- WO-A-97/49378
- FR-A- 2 766 178

## Description

L'invention a pour objet de nouvelles pyrazolo[1,5-a]pyrimidines comportant au moins un groupement cationique Z, Z étant choisi parmi des chaînes aliphatiques quatemisées, des chaînes aliphatiques comportant au moins un cycle saturé quaternisé, et des chaînes aliphatiques comportant au moins un cycle insaturé quaternisé, leur utilisation à titre de base d'oxydation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir, de façon totalement inattendue et surprenante, que de nouvelles pyrazolo[1,5-a]pyrimidines de formule (I) ci-après définies comportant au moins un groupement cationique Z, Z étant choisi parmi des chaînes aliphatiques quaternisées, des chaînes aliphatiques comportant au moins un cycle saturé quatemisé, et des chaînes aliphatiques comportant au moins un cycle insaturé quatemisé, non seulement conviennent pour une utilisation comme précurseurs de colorant d'oxydation, mais en outre qu'elles permettent d'obtenir des compositions tinctoriales conduisant à des colorations puissantes, dans une large palette de couleurs, et présentant d'excellentes propriétés de résistances aux différents traitements que peuvent subir les fibres kératiniques.

Ces découvertes sont à la base de la présente invention.

L'invention a donc pour premier objet de nouveaux composés de formule (I) suivante, et leurs sels d'addition avec un acide : 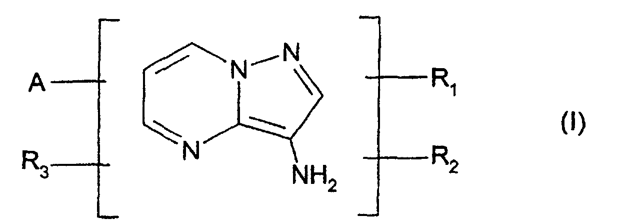 dans laquelle :
- R₁, R₂ et R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical hydroxyle ; un radical nitro ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un radical amino ; un radical N-alkyl(C₁-C₆)amino ; un radical N,N-dialkyl(C₁-C₆)amino (les deux substituants alkyle pouvant former un cycle à 5 ou 6 chaînons) ; un radical N-hydroxyalkyl(C₁-C₆)amino ; un radical N,N-bis(hydroxyalkyl(C₁-C₆))amino ; un radical N-polyhydroxyalkyl(C₂-C₆)amino ; un radical N,N-bis(polyhydroxyalkyl (C₂-C₆))amino ; un radical aminoalkyl(C₁-C₆)amino dans lequel le groupement amino terminal est non substitué ou substitué par un ou deux radicaux alkyle en C₁-C₆, lesdits radicaux alkyle pouvant former un cycle saturé ou non à 5 ou 6 chaînons ; un groupe amino protégé par un radical alkyl(C₁-C₆)carbonyle, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, formyle, ou par un groupement Z ;
- R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)sulfonyle, ou par un groupement Z ;
- A représente un groupement -NR₄R₅ ou un radical hydroxyle ;
- R₄ et R₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, alkyl(C₁-C₆)sulfonyle, formyle, trifluoroalkyl(C₁-C₆)carbonyle, ou par un groupement Z ;
   un et un seul des radicaux R₄ et R₅ peut également représenter un radical alkyl(C₁-C₆)carbonyle ; formyle ; trifluoroalkyl(C₁-C₆)carbonyle ; aminoalkyl(C₁-C₆)carbonyle ; N-alkyl(C₁-C₆)aminoalkyl(C₁-C₆)carbonyle ; N-N-dialkyl(C₁-C₆)aminoalkyl(C₁-C₆)carbonyle ;
- Z est choisi parmi les groupements cationiques insaturés de formules (II) et (III) suivantes, et les groupements cationiques saturés de formule (IV) suivante : 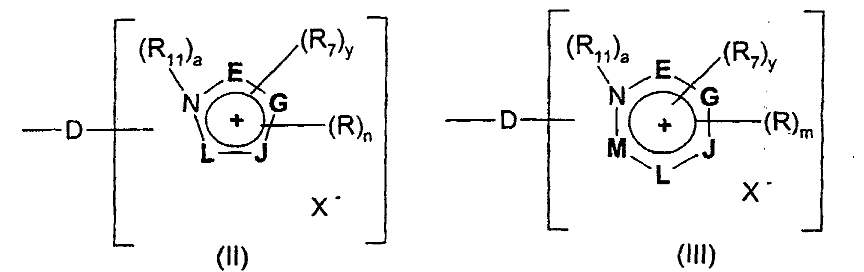 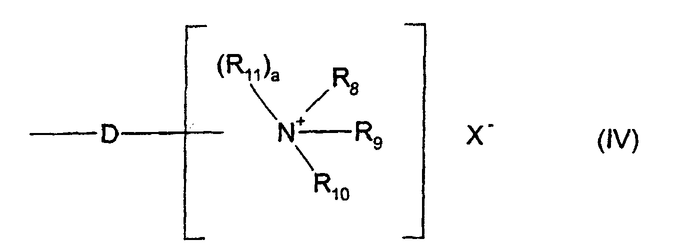
dans lesquelles :
- D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
- les sommets E, G, J, L et M, identiques ou différents, forment avec l' azote du cycle des cycles choisis parmi les cycles pyrrolique, imidazolique, pyrazolique, oxazolique, thiazolique, triazolique, pyridinique, pyrimidinique, pyrazinique, oxazinique et triazinique.
- n est un nombre entier compris entre 0 et 4 inclusivement ;
- m est un nombre entier compris entre 0 et 5 inclusivement ;
- les radicaux R, identiques ou différents, représentent un atome d'halogène ; un radical hydroxyle ; un radical alkyle en C₁-C₆ ; un radical monohydroxyalkyle en C₁-C₆ ; un radical polyhydroxyalkyle en C₂-C₆ ; un radical nitro ; un radical cyano ; un radical cyanoalkyle en C₁-C₆ ; un radical alcoxy en C₁-C₆ ; un radical trialkyl(C₁-C₆)silanealkyle en C₁-C₆ ; un radical amido ; un radical aldéhydo ; un radical carboxyle ; un radical alkylcarbonyle en C₁-C₆ ; un radical thio ; un radical thioalkyle en C₁-C₆ ; un radical alkyl(C₁-C₆)thio ; un radical amino ; un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
- R₇ représente un radical alkyle en C₁-C₆ ; un radical monohydroxyalkyle en C₁-C₆ ; un radical polyhydroxyalkyle en C₂-C₆ ; un radical cyanoalkyle en C₁-C₆ ; un radical trialkyl(C₁-C₆)silanealkyle en C₁-C₆ ; un radical alcoxy(C₁-C₆)alkyle en C₁-C₆ ; un radical carbamylalkyle C₁-C₆ ; un radical alkyl(C₁-C₆)carboxyalkyle en C₁-C₆ ; un radical benzyle ;
- R₈, R₉ et R₁₀, identiques ou différents, représentent un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical cyanoalkyle en C₁-C₆ ; un radical aryle ; un radical benzyle ; un radical amidoalkyle en C₁-C₆ ; un radical trialkyl(C₁-C₆)silanealkyle en C₁-C₆ ou un radical aminoalkyle en C₁-C₆ dont l'amine est protégée par un radical alkyl(C₁-C₆)carbonyle, amido, carboxyle ou alkyl(C₁-C₆)sulfonyle ; deux des radicaux R₈, R₉ et R₁₀ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
- R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- a et y sont des nombres entiers égaux à 0 ou 1 ; avec les conditions suivantes :
   - dans les groupements cationiques insaturés de formule (II) :
      - lorsque a = 0, le bras de liaison D est rattaché à l'atome d'azote,
      - lorsque a = 1, le bras de liaison D est rattaché à l'un des sommets E, G, J ou L,
      - y ne peut prendre la valeur 1 que :
         1) lorsque les sommets E, G, J et L représentent simultanément un atome de carbone, et que le radical R₇ est porté par l'atome d'azote du cycle insaturé ; ou bien
         2) lorsqu'au moins un des sommets E, G, J et L représente un atome d'azote sur lequel le radical R₇ est fixé ;
   - dans les groupements cationiques insaturés de formule (III) :
      - lorsque a = 0, le bras de liaison D est rattaché à l'atome d'azote,
      - lorsque a = 1, le bras de liaison D est rattaché à l'un des sommets E, G, J, L ou M,
      - y ne peut prendre la valeur 1 que lorsqu'au moins un des sommets E, G, J, L et M représente un atome divalent, et que le radical R₇ est porté par l'atome d'azote du cycle insaturé ;
   - dans les groupements cationiques de formule (IV) :
      - lorsque a = 0, alors le bras de liaison D est rattaché à l'atome d'azote portant les radicaux R₈ à R₁₀,
      - lorsque a = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
- X représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
étant entendu que le nombre de groupement cationique Z est au moins égal à 1.

Comme indiqué précédemment, les colorations obtenues avec la composition de teinture d'oxydation conforme à l'invention sont puissantes et permettent d'atteindre une large palette de couleurs. Elles présentent de plus d'excellentes propriétés de résistance vis à vis de l'action des différents agents extérieurs (lumière, intempéries, lavage, ondulation permanente, transpiration, frottements). Ces propriétés sont particulièrement remarquables notamment en ce qui concerne la résistance des colorations obtenues vis à vis de l'action de la lumière, des lavages, de l'ondulation permanente et de la transpiration.

Dans la formule (I) ci-dessus les radicaux alkyle et alcoxy peuvent être linéaires ou ramifiés.

Parmi les cycles des groupements insaturés Z de formule (II) ci-dessus, on peut notamment citer à titre d'exemple les cycles pyrrolique, imidazolique, pyrazolique, oxazolique, thiazolique et triazolique.

Parmi les cycles des groupements insaturés Z de formule (III) ci-dessus, on peut notamment citer à titre d'exemple les cycles pyridinique, pyrimidinique, pyrazinique, oxazinique et triazinique.

Parmi les composés de formule (I) ci-dessus, on peut notamment citer :
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le chlorure de 3-[(3-amino-pyrazolo[1,5-a]pyrimidin-7-ylcarbamoyl)-méthyl]-1-méthyl-3H-imidazol-1-ium,
- le méthyl sulfaté de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le méthyl sulfate de 2-[(3-amino-pyrazolo[1,5-a]pyrimidin-7-ylamino)-méthyl]-1,3-diméthyl-3H-imidazol-1-ium,
- le méthyl sulfate de 3-[(3-amino-pyrazolo[1,5-a]pyrimidin-7-ylamino)-méthyl]-1-méthyl-pyridinium,
- le méthyl sulfate de 3-[(3-amino-pyrazolo[1,5-a]pyrimidin-7-ylamino)-méthyl]-1-méthyl-pyridinium,
- le méthyl sulfate de 2-(3,7-diamino-5-méthyl-pyrazolo[1,5-a]pyrimidin-6-ylméthyl)-1,3-diméthyl-3H-imidazol-1-ium,
- le méthyl sulfate de 2-(3-amino-7-hydroxy-5-méthyl-pyrazolo[1,5-a]pyrimidin-6-ylméthyl)-1,3-diméthyl-3H-imidazol-1-ium,
- le méthyl sulfate de 2-(3,7-diamino-pyrazolo[1,5-a]pyrimidin-2-yl)-1-méthyl-pyridinium,
- le chlorure de [3-(3-amino-5-méthyl-pyrazolo[1,5-a]pyrimidin-7-ylamino)-propyl]-triméthyl-ammonium,
- le méthyl sulfate de [3-(3-amino-5-méthyl-pyrazolo[1,5-a]pyrimidin-7-ylamino)-propyl]-triméthyl-ammonium,
- le chlorure de 1-[3-(3-amino-5-méthyl-pyrazolo[1,5-a]pyrimidin-7-ylamino)-propyl]-1-méthyl-pipéridinium,
- le méthyl sulfate de 1-[3-(3-amino-5-méthyl-pyrazolo[1,5-a]pyrimidin-7-ylamino)-propyl]-1-méthyl-pipéridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition avec un acide.

Parmi ces composés de formule (I), on préfère plus particulièrement :
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le chlorure de 3-(3-amino-7-hydroxy-5-méthyl-pyrazolo[1,5-a]pyrimidin-6-ylméthyl)-1-méthyl-pyridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition avec un acide.

Les composés de formule (I) conformes à l'invention peuvent être facilement obtenus, selon des méthodes bien connues de l'état de la technique :
- soit par réduction des composés nitrés ou nitrosés cationiques correspondants. Dans ce cas, la réduction en amine aromatique primaire correspondante est effectuée selon des méthodes classiques (J. Lehmman dans "Houben-Weyl, "Methoden der Organischen Chemie", Band IV/1c : Reduktion I page 491 à 537, 1980). Les méthodes préférées selon l'invention font intervenir des métaux comme Zn, Sn, ou Fe en milieu acide comme l'acide chlorhydrique aqueux, ou l'acide acétique aqueux en présence ou non d'un co-solvant comme le méthanol, l'éthanol ou le tétrahydrofurane. L'hydrogénation catalytique est une méthode de réduction préférée selon l'invention. Cette hydrogénation catalytique utilise des métaux comme le palladium, le platine ou le nickel. On préfère encore plus particulièrement le palladium sur charbon ou le nickel de Raney, ou bien encore des oxydes comme PtO₂ dans des solvants comme le méthanol, l'éthanol, le tétrahydrofurane ou l'acétate d'éthyle en présence ou non d'un acide comme par exemple l'acide acétique. Ces réductions catalytiques peuvent aussi être effectuées avec de l'acide formique en présence d'une trialkylamine comme la triéthylamine ou avec du formiate d'ammonium à la place de l'hydrogène gazeux. (S. Ram, R.E. Ehrenkaufer, Synthesis,1988,91) ;
- soit par réduction des composés azoïques cationiques correspondants (coupure réductrice). La réduction en amine aromatique primaire correspondante est effectuée selon des méthodes classiques (J. Lehmman dans "Houben-Weyl, "Methoden der Organischen Chemie", Band IV/1c: Reduktion I page 551 à 553, 1980 ; E.C. Taylor & Coll., J. Amer. Chem. Soc, 80, 421, 1958).

Cette étape de réduction (obtention d'une amine aromatique primaire) qui confère au composé synthétisé son caractère de composé oxydable (de bas d'oxydation) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quatemisation) et terminer par le "déprotection" (en général en milieu acide) de la fonction amine.

Lorsque la synthèse est terminée, les composés de formule (I) conformes à l'invention peuvent, le cas échéant, être récupérés par des méthodes bier connues de l'état de la technique telles que la cristallisation, la distillation.

Un autre objet de l'invention est l'utilisation des composés de formules (I) conformes à l'invention à titre de base d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 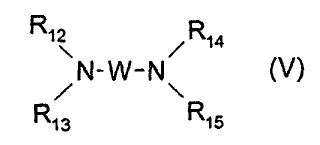 dans laquelle W est un reste propylène éventuellement substitué par un groupement hydroxyle ou un radical alkyle en C₁-C₆ ; R₁₂, R₁₃, R₁₄ et R₁₅, identiques ou différents, représentent un atome d'hydrogène, un radical alkyle en C₁-C₆ ou hydroxyalkyle en C₁-C₆.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques différentes des composés de formule (I).

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques non cationiques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES DE PREPARATION

### EXEMPLE DE PREPARATION 1 : Synthèse du chlorure de 3-[3-(3-amino-5-méthyl-pyrazolo[1,5-a]pyrimidin-7-ylamino)-propyl]-1-(2-hydroxyéthyl)-3H-imidazol-1-ium, dichlorhydrate

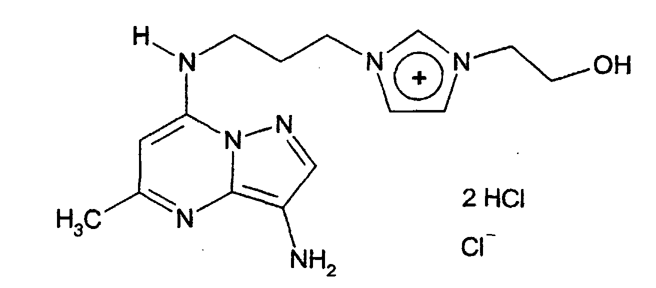

### A) Préparation du 3-nitro-5-méthyl-pyrazolo-[1,5-a]-pyrimidin-7-ol

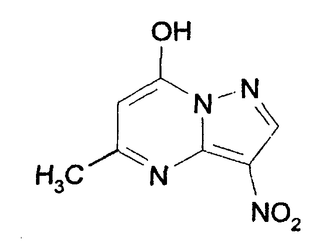

On a introduit, dans un ballon tricol de 500 cc équipé d'une agitation magnétique, d'un thermomètre et d'un réfrigérant, 50 g de chlorhydrate de 4-nitro-2H-pyrazol-3-ylamine (préparé selon H. Dorn et H. Dilcher, Liebigs Ann.Chem., 707, 141, 1967) et 60 g d'acétoacétate d'éthyle dans 160 cc d'acide acétique. On a porté le milieu réactionnel au reflux pendant 12 heures. On a filtré vers 90°C le précipité qui s'est formé. On l'a rincé à l'éther diisopropylique et séché sous vide sur anhydride phosphorique. On a obtenu 50 g de 3-nitro-5-méthyl-pyrazolo-[1,5-a]-pyrimidin-7-ol sous forme de cristaux jaunes (rendement = 84,5% ; point de fusion = 290°C avec décomposition), dont l'analyse élémentaire calculée pour C₇ H₆ N₄ O₃ (PM=194,15 g) était :

| % | C | H | N | O |
|---|---|---|---|---|
| Calculée | 43,31 | 3,12 | 28,86 | 24,72 |
| Trouvée | 43,12 | 3,11 | 28,77 | 24,65 |

### b) Préparation de la 7-chloro-5-méthyl-3-nitro-pyrazolo-[1,5-a]-pyrimidine

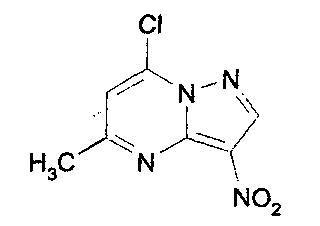

Dans un tricol de 500 cc équipé d'une agitation magnétique, d'un thermomètre et d'un réfrigérant, on a introduit 230 cc d'oxychlorure de phosphore, 15,4 g de N,N-diméthylaniline et 23,3 g de 3-amino-5-méthyl-pyrazolo-[1,5-a]-pyrimidin-7-ol. Le milieu réactionnel a été porté au reflux pendant 2h30. Après évaporation de l'oxychlorure de phosphore sous pression réduite, on a obtenu une huile verte très visqueuse à laquelle on a ajouté environ 400 g de glace. Un solide brun a précipité. Après 30 minutes d'agitation, on a filtré le précipité et on l'a rincé à l'éther de pétrole puis à l'éther diisopropylique. Après séchage sous vide sur anhydride phosphorique, on a obtenu 21,4 g de 7-chloro-5-methyl-3-nitro-pyrazolo-[1,5-a]-pyrimidine sous forme d'un solide brun avec un rendement de 83,9%.

### c) Préparation de la (3-imidazol-1-yl-propyl)-(5-méthyl-3-nitro-pyrazolo[1,5-a]pyrimidin-7-yl)-amine

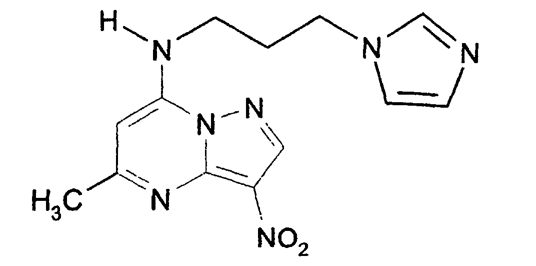

Dans un ballon tricol de 100 cc équipé d'une agitation magnétique, d'un thermomètre, d'une ampoule à addition et d'un réfrigérant, on a introduit 2,88 g de 3-imidazol-1-yl-propylamine et 2,33 g de triéthylamine dans 20 cc de dioxane. On a additionné goutte à goutte 4,5 g de 7-chloro-5-méthyl-3-nitro-pyrazolo-[1,5-a]-pyrimidine en solution dans 20 cc de dioxane et 5 cc de diméthylformamide. Après 2 heures d'agitation à température ambiante, on a filtré le précipité. On l'a rincé à l'ether diisopropylique et séché sous vide. On a obtenu 7,2 g de produit brut. On l'a repris au reflux dans 28 cc d'eau, puis filtré à température ambiante. On a répété cette opération une deuxième fois. On a rincé le produit à l'éthanol et à l'ether diisopropylique. On a obtenu 4,1 g de (3-imidazol-1-yl-propyl)-(5-méthyl-3-nitro-pyrazolo[1,5-a]pyrimidin-7-yl)-amine sous forme d'une poudre beige après séchage sous vide sur anhydride phosphorique, avec un rendement de 65%.

### d) Préparation du chlorure de 1-(2-hydroxy-éthyl)-3-[3-(5-méthyl-3-nitro-pyrazolo[1,5-a]pyrimidin-7-ylamino)-propyl]-3H-imidazol-1-ium

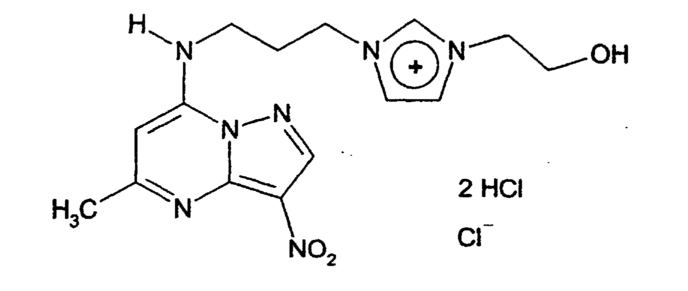

Dans un ballon tricol de 25 cc équipé d'une agitation magnétique, d'un thermomètre et d'un réfrigérant, on a introduit 3 g de (3-imidazol-1-yl-propyl)-(5-méthyl-3-nitro-pyrazolo[1,5-a]pyrimidin-7-yl)-amine et 10 g de 2-chloroéthanol. On a porté le milieu au reflux pendant 6 heures. On a versé le milieu réactionnel sur 160 cc d'acétate d'éthyle et porté au reflux. On a filtré à température ambiante le précipité. On a obtenu 3,8 g de chlorure de 1-(2-hydroxyéthyl)-3-[3-(5-méthyl-3-nitro-pyrazolo[1,5-a]pyrimidin-7-ylamino)-propyl]-3H-imidazol-1-ium (poudre beige) après séchage sous vide sur anhydride phosphorique.

### e) Préparation du chlorure de 3-[3-(3-amino-5-méthyl-pyrazolo[1,5-a]pyrimidin-7-ylamino)-propyl]-1-(2-hydroxyéthyl)-3H-imidazol-1-ium dichlorhydrate

Dans un hydrogénateur de 250 cc, on a introduit 3,5 g de 1-(2-hydroxyéthyl)-3-[3-(5-méthyl-3-nitro-pyrazolo[1,5-a]pyrimidin-7-ylamino)-propyl]-3H-imidazol-1-ium dans 150 cc d'éthanol puis 0,39 g de palladium à 5% sur charbon (contenant 50% d'eau). On a introduit entre 11 et 12 bars de pression d'hydrogène dans le réacteur et porté le milieu réactionnel à 60°C. Après 4 heures de réaction, on a filtré le catalyseur sur célite et fait passer un courant d'acide chlorhydrique gazeux à travers le filtrat. On a versé le milieu réactionnel sur 100 cc d'ether diisopropylique. Après agitation, on a filtré le précipité. On l'a lavé à l'ether diisopropylique et séché sous vide sur anhydride phosphorique. On a obtenu 3,3 g d'un produit très hygroscopique. On fait une solution aqueuse de ce produit à 3% que l'on a lyophilisée. On a repris le solide obtenu au reflux de 30 cc d'éthanol absolu. On obtient ainsi 2,25 g de chlorure de 3-[3-(3-amino-5-méthyl-pyrazolo[1,5-a]pyrimidin-7-ylamino)-propyl]-1-(2-hydroxy-éthyl)-3H-imidazol-1-ium sous forme de dichlorhydrate après séchage sous vide sur anhydride phosphorique avec un rendement de 85% et dont l'analyse calculée pour C₁₅ H₂₂ N₇ O Cl, 2HCl (PM =424,76 g) était :

| % | C | H | N | O | Cl |
|---|---|---|---|---|---|
| Calculée | 42,42 | 5,70 | 23,08 | 3,77 | 25,04 |
| Trouvée | 40,28 | 6,19 | 21,40 | 7,99 | 24,14 |
| Calculée avec 1 mole d'eau | 40,69 | 5,92 | 22,14 | 7,23 | 24,02 |

### EXEMPLE DE PREPARATION 2 : Synthèse du chlorure de 3-(3-amino-7-hydroxy-5-méthyl-pyrazolo[1,5-a]pyrimidin-6-ylméthyl)-1-(2-hydroxyéthyl)-pyridinium, chlorhydrate

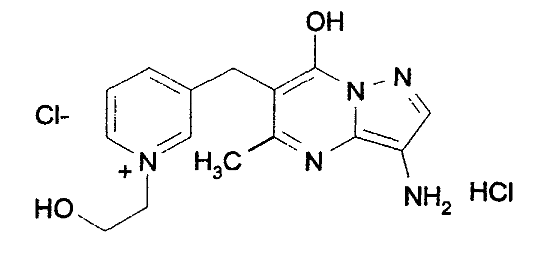

### a) Préparation du méthyl ester de l'acide 3-oxo-2-pyridin-3-ylméthyl-butyrique

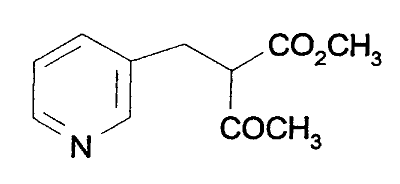

Dans un réacteur à hydrogénation de 300 cc, on a introduit 25 g du méthyl ester de l'acide 2-acétyl-3-pyridin-3-yl-acrylique (préparé selon I. ADACHI & coll., Chem. Pharm. Bull. 35(8), 3235, 1987), 200 cc d'éthanol et 5,25 g de palladium sur charbon à 5% (contenant 50% d'eau). On a introduit une pression d'hydrogène de 6 bars et effectué la réduction à température ambiante. On a traité le milieu réactionnel lorsqu'il n'y avait plus d'absorption d'hydrogène. On a filtré le catalyseur et évaporé le solvant. On a obtenu 24 g de produit brut qu'on a traité avec 200 cc d'ether diéthylique. On a filtré le précipité blanc et on a évaporé le solvant. On a obtenu 20 g de méthyl ester de l'acide 3-oxo-2-pyridin-3-ylméthyl-butyrique sous forme d'une huile marron, avec un rendement de 79%.

### b) Préparation du chlorhydrate de 5-méthyl-3-nitro-6-pyridin-3-ylméthyl-pyrazolo[1,5-a] pyrimidin-7-ol

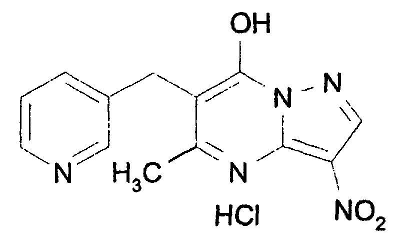

On a introduit, dans un ballon tricol de 2 litres équipé d'une agitation magnétique, d'un thermomètre et d'un réfrigérant, 89 g de chlorhydrate de 4-nitro-2H-pyrazol-3-ylamine (préparé selon H. Dom et H. Dilcher, Liebigs Ann.Chem., 707, 141, 1967) et 112 g de méthyl ester de l'acide 3-oxo-2-pyridin-3-ylméthyl-butyrique obtenu ci-dessus à l'étape précédente dans 1120 cc d'acide acétique. On a porté le milieu réactionnel au reflux pendant 5 heures. On a filtré à température ambiante le précipité qui s'est formé. On l'a rincé à l'éther diisopropylique et séché sous vide sur anhydride phosphorique. On a obtenu 120 g de produit brut. On l'a recristallisé dans un mélange eau/acétone (1/25). On a obtenu 77 g de chlorhydrate de 5-méthyl-3-nitro-6-pyridin-3-ylméthyl-pyrazolo[1,5-a] pyrimidin-7-ol sous forme de cristaux jaunes (rendement = 50%), dont l'analyse élémentaire calculée pour C₁₃ H₁₁ N₅ O₃ . HCI était :

| % | C | H | N | O | Cl |
|---|---|---|---|---|---|
| Calculée | 48,53 | 3,76 | 21,77 | 14,92 | 11,02 |
| Trouvée | 48,31 | 3,82 | 21,89 | 14,23 | 11,75 |

### c) Préparation du 5-méthyl-3-nitro-6-pyridin-3-ylméthyl-pyrazolo[1,5-a] pyrimidin-7-ol

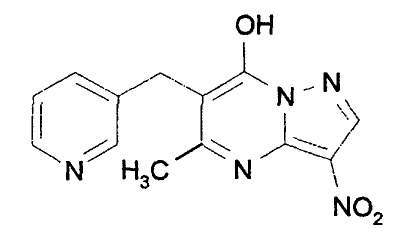

Dans un Erlenmeyer de 500 cc équipé d'une agitation magnétique, on a introduit 250 cc d'eau et 10,6 g d'ammoniaque à 20%. On a additionné 20 g de chlorhydrate de 5-méthyl-3-nitro-6-pyridin-3-ylm"thyl-pyrazolo[1,5-a] pyrimidin-7-ol par portions solides. On a laissé pendant 3 heures sous agitation à température ambiante On a filtré le solide obtenu puis lavé avec 100 cc d'eau, puis avec de l'ether diisopropylique. On a séché le produit sur anhydride phosphorique. On a obtenu 16 g de 5-méthyl-3-nitro-6-pyridin-3-ylméthyl-pyrazolo[1,5-a] pyrimidin-7-ol avec un rendement de 90%.

### d) Préparation du chlorure de 1-(2-hydroxy-éthyl)-3-(7-hydroxy-5-méthyl-3-nitro-pyrazolo[1,5-a]pyrimidin-6-ylméthyl)-pyridinium

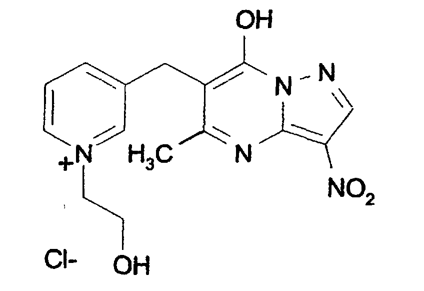

Dans un ballon tricol de 250 cc équipé d'une agitation magnétique, d'un thermomètre et d'un réfrigérant, on a introduit 10 g de 5-méthyl-3-nitro-6-pyridin-3-ylméthyl-pyrazolo[1,5-a] pyrimidin-7-ol, obtenu ci-dessus à l'étape précédente, et 100 cc de 2-chloroéthanol. On a porté le milieu au reflux pendant 5 heures. On a évaporé le solvant puis on a traité le produit avec de l'éthanol. On a filtré à température ambiante le précipité. On a obtenu 10 g de produit brut. On l'a recristallisé dans l'acide acétique. On a obtenu 6,7 g de chlorure de 1-(2-hydroxy-éthyl)-3-(7-hydroxy-5-méthyl-3-nitro-pyrazolo[1,5-a]pyrimidin-6-ylméthyl)-pyridinium après séchage sous vide sur anhydride phosphorique (Rendement = 52%) et dont l'analyse élémentaire calculée pour C₁₅ H₁₆ N₅ O₄. Cl. avec 0,28 moles d'acide acétique était :

| % | C | H | N | O | Cl |
|---|---|---|---|---|---|
| Calculée | 48,80 | 4,47 | 18,29 | 19,07 | 9,28 |
| Trouvée | 47,69 | 4,56 | 18,26 | 18,85 | 9,51 |

### e) Préparation du chlorure de 3-(3-amino-7-hydroxy-5-méthyl-pyrazolo[1,5-a]pyrimidin-6-ylméthyl)-1-(2-hydroxy-éthyl)-pyridinium, chlorhydrate

Dans un hydrogénateur de 500 cc, on a introduit 2 g de chlorure de 1-(2-hydroxy-éthyl)-3-(7-hydroxy-5-méthyl-3-nitro-pyrazolo[1,5-a]pyrimidin-6-ylméthyl)-pyridinium obtenu ci-dessus à l'étape précédente dans 200 cc d'acide acétique puis 0,6 g de palladium à 5% sur charbon (contenant 50% d'eau). On a introduit 8 bars de pression d'hydrogène dans le réacteur et porté le milieu réactionnel à 50°C. Après 3 heures de réaction, on a filtré le catalyseur sur célite. On a évaporé le solvant et on a repris le produit brut obtenu dans 10 cc d'éthanol chlorhydrique 7M. On a filtré le précipité. On l'a lavé à l'ether diisopropylique et séché sous vide sur anhydride phosphorique. On a obtenu 2,7 g de chlorure de 3-(3-amino-7-hydroxy-5-méthyl-pyrazolo[1,5-a]pyrimidin-6-ylméthyl)-1-(2-hydroxy-éthyl)-pyridinium (produit très hygroscopique) sous forme de chlorhydrate après séchage sous vide sur anhydride phosphorique avec un rendement de 75% et dont l'analyse calculée pour C₁₅ H₁₈ N₅ O₂ Cl, HCI était :

| % | C | H | N | O | Cl |
|---|---|---|---|---|---|
| Calculée | 48,40 | 5,14 | 18,81 | 8,60 | 19,05 |
| Trouvée | 47,04 | 5,25 | 17,59 | 10,26 | 18,40 |
| Calculée avec 0,5 mole d'eau | 47,20 | 5,24 | 18,34 | 10,48 | 18,61 |

### EXEMPLES D'APPLICATION

### EXEMPLES 1 à 7 DE TEINTURE EN MILIEU BASIQUE

On a préparé les compositions tinctoriales suivantes (teneurs en grammes) :

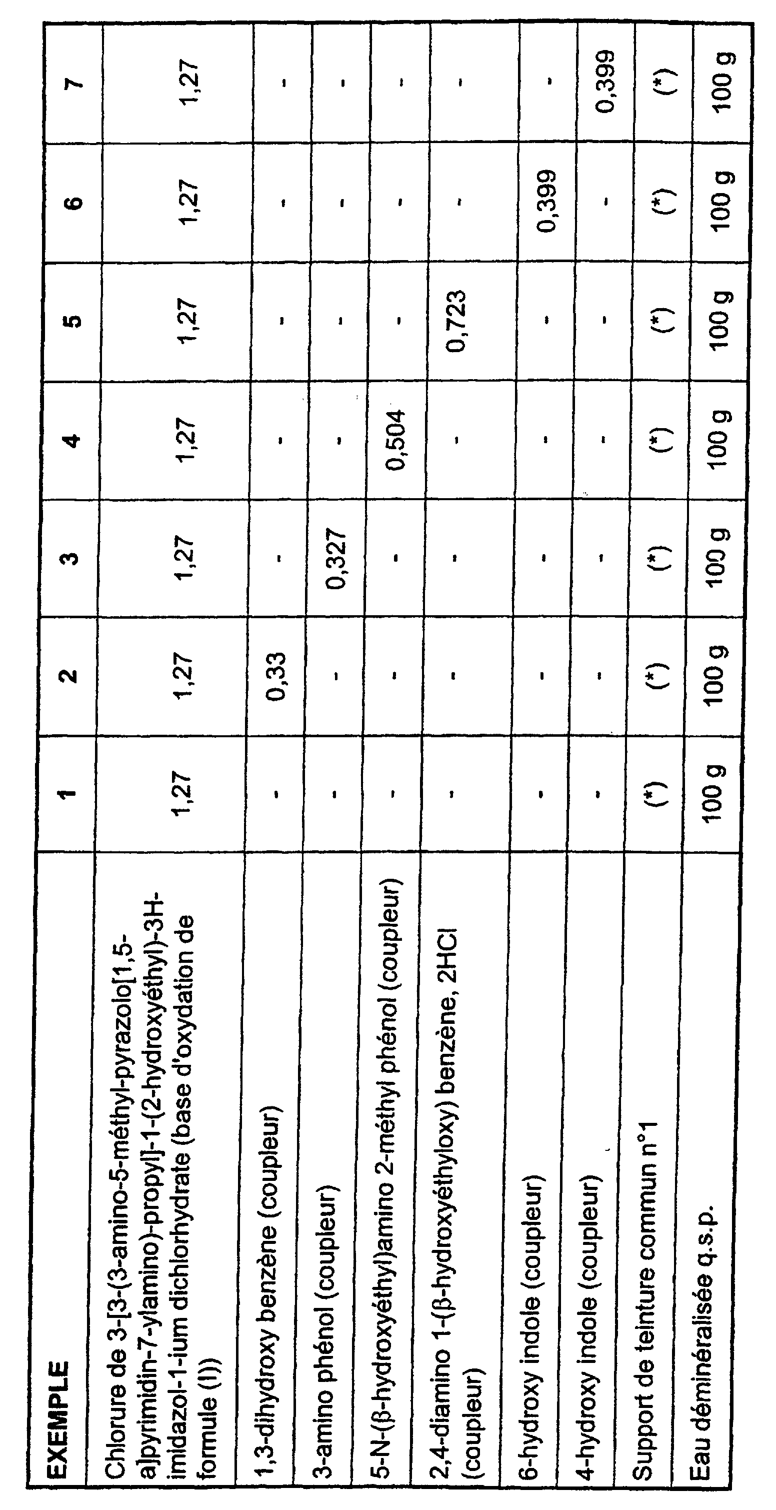

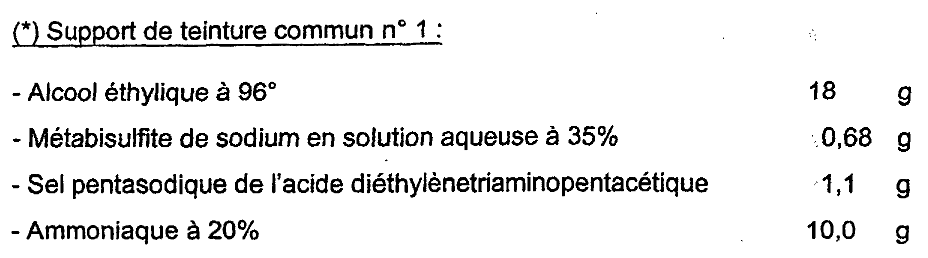

Au moment de l'emploi, on a mélangé poids pour poids chacune des compositions tinctoriales ci-dessus avec une solution de peroxyde d'hydrogène à 20 volumes (6 % en poids) de pH 3. Le mélange obtenu a été appliqué sur des mèches de cheveux gris permanentés à 90 % de blancs pendant 30 minutes. Les mèches ont ensuite été rincés, lavés avec un shampooing standard, rincées à nouveau puis séchées.
Les nuances obtenues figurent dans le tableau ci-après :

| **EXEMPLE** | **pH de teinture** | **Nuance obtenue** |
|---|---|---|
| **1** | 10 ± 0,2 | Blond foncé irisé |
| **2** | 10 ± 0,2 | Rouge cuivré |
| **3** | 10 ± 0,2 | Acajou violine |
| **4** | 10 ± 0,2 | Cuivré rouge |
| **5** | 10 ± 0,2 | Châtain clair irisé violacé profond |
| **6** | 10 ± 0,2 | Châtain naturel |
| **7** | 10 ± 0,2 | Irisé rouge profond |

### EXEMPLES 8 à 14 DE TEINTURE EN MILIEU NEUTRE

On a préparé les compositions tinctoriales suivantes (teneurs en grammes) :

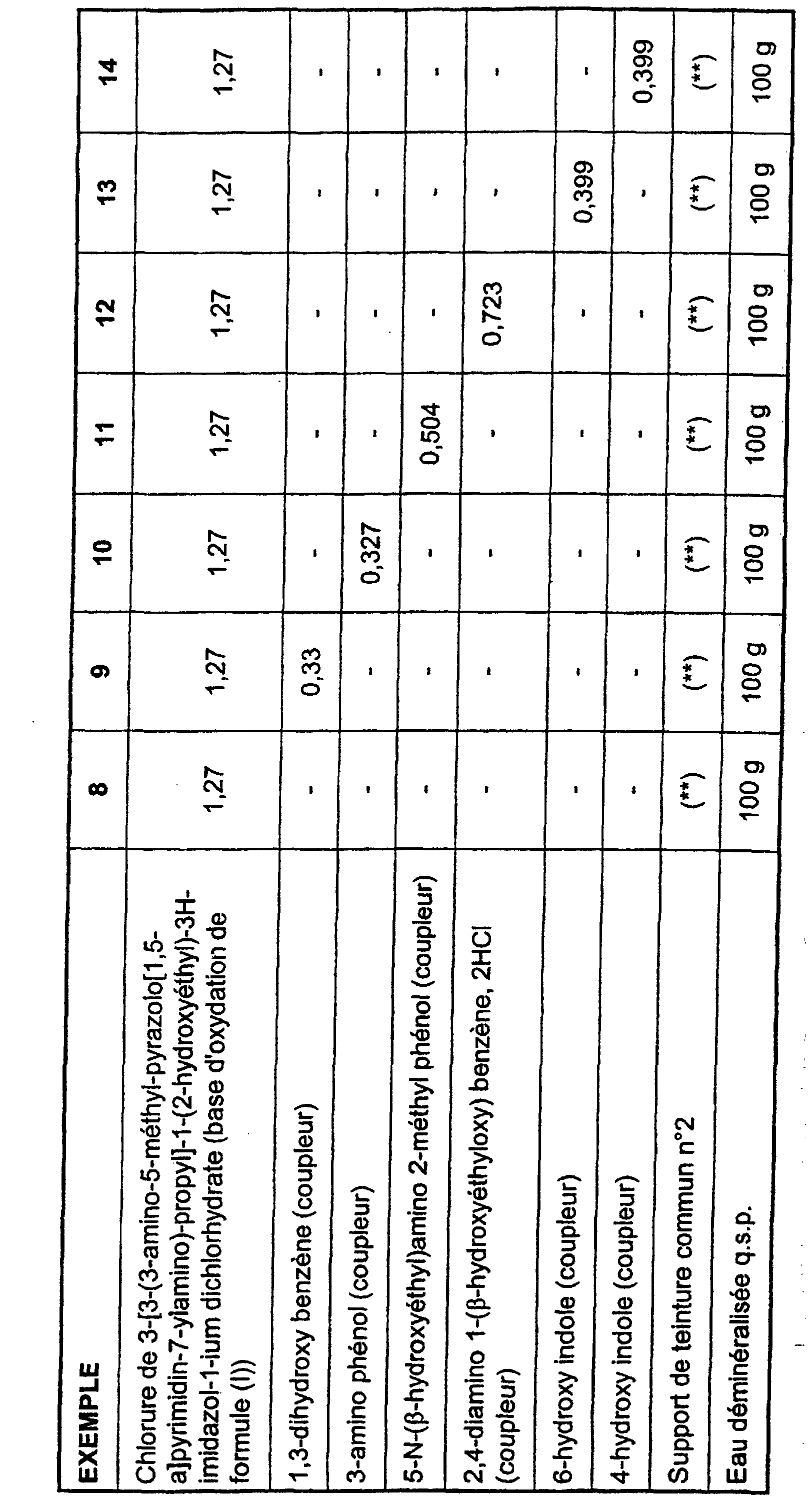

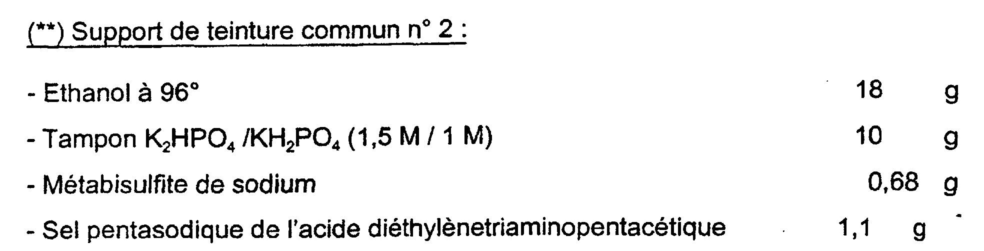

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.
Les nuances obtenues figurent dans le tableau ci-après :

| **EXEMPLE** | **pH de teinture** | **Nuance obtenue** |
|---|---|---|
| **8** | 5,7 ± 0,2 | Blond irisé cuivré |
| **9** | 5,7 ± 0,2 | Blond clair beige cendré |
| **10** | 5,7 ± 0,2 | Blond foncé marron cuivré |
| **11** | 5,7 ± 0,2 | Blond foncé cuivré |
| **12** | 5,7 ± 0,2 | Rouge violine |
| **13** | 5,7 ± 0,2 | Blond foncé naturel cendré |
| **14** | 5,7 ± 0,2 | Violine rouge |

## Revendications

1. Composés de formule (I) suivante, et leurs sels d'addition avec un acide : 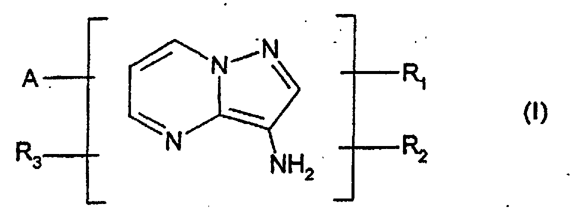 dans laquelle :
• R₁, R₂ et R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₈)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dlalkyl(C₁-C₆)carbamylalkyle(C₁-C₈) ; un radical alkyle en C₁-C₆ ; un radical hydroxyle ; un radical nitro ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un radical amino ; un radical N-alkyl(C₁-C₆)amino ; un radical N,N-dialkyl(C₁-C₆)amino (les deux substituants alkyle pouvant former un cycle à 5 ou 6 chaînons) ; un radical N-hydroxyalkyl(C₁-C₆)amino ; un radical N,N-bis(hydroxyalkyl(C₁-C₆))amino ; un radical-N-polyhydroxyalkyl(C₂-C₆)amino ; un radical N,N-bis(polyhydroxyalkyl (C₂-C₆))amino ; un radical aminoalkyl(C₁-C₈)amino dans lequel le groupement amino terminal est non substitué ou substitué par un ou deux radicaux alkyle en C₁-C₆, lesdits radicaux alkyle pouvant former un cycle saturé ou non à 5 ou 6 chaînons ; un groupe amino protégé par un radical alkyl(C₁-C₆)carbonyle, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, formyle, ou par un groupement Z;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆); un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)sulfonyle, ou par un groupement Z ;
• A représente un groupement -NR₄R₅ ou un radical hydroxyle ;
• R₄ et R₅, identiques ou différents, représentent un atome d'hydrogène ; un groupement Z ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, alkyl(C₁-C₆)sulfonyle, formyle, trifluoroalkyl(C₁-C₆)carbonyle, ou par un groupement Z ;
un et un seul des radicaux R₄ et R₅ peut également représenter un radical alkyl(C₁-C₆)carbonyle ; formyle ; trifluoroalkyl(C₁-C₆)carbonyle ; aminoalkyl(C₁-C₆)carbonyle; N-alkyl(C₁-C₆)aminoalkyl(C₁-C₆)carbonyle ; N-N-dialkyl(C₁-C₆)aminoalkyl(C₁-C₆)carbonyle ;
• Z est choisi parmi les groupements cationiques insaturés de formules (II) et (III) suivantes, et les groupements cationiques saturés de formule (IV) suivante : 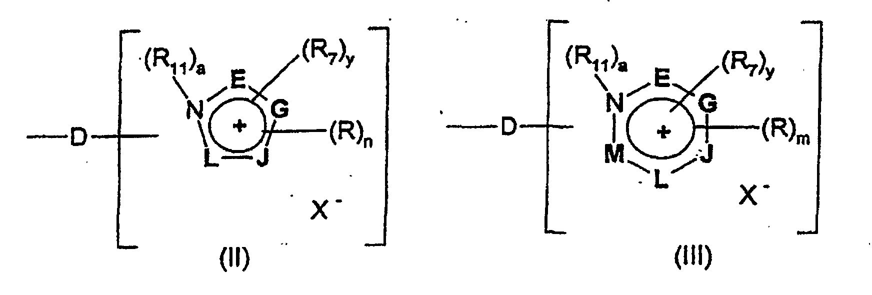 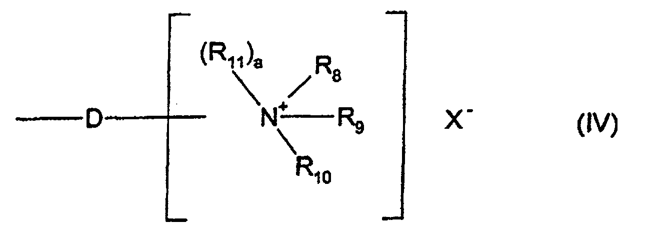
dans lesquelles :
• D est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une
ou plusieurs fonctions cétone ;
• les sommets E, G, J, L et M, identiques ou différents, forment avec l'azote du cycle des cycles choisis parmi les cycles pyrrolique, imidazolique, pyrazolique, oxazolique, thiazolique et triazolique, pyridinique, pyrimidinique, pyrazinique, oxazinique et triazinique,
• n est un nombre entier compris entre 0 et 4 inclusivement ;
• m est un nombre entier compris entre 0 et 5 inclusivement ;
• les radicaux R, identiques ou différents, représentent ; un atome d'halogène ; un radical hydroxyle ; un radical alkyle en C₁-C₆ ; un radical monohydroxyalkyle en C₁-C₆ ; un radical polyhydroxyalkyle en C₂-C₆ ; un radical nitro ; un radical cyano ; un radical cyanoalkyle en C₁-C₆ ; un radical alcoxy en C₁-C₆ ; un radical trialkyl(C₁-C₆)silanealkyle en C₁-C₆ ; un radical amido ; un radical aldéhydo ; un radical carboxyle ; un radical alkylcarbonyle en C₁-C₆ ; un radical thio ; un radical thioalkyle en C₁-C₆ ; un radical alkyl(C₁-C₆)thio ; un radical amino ; un radical amino protégé par un radical alkyl(C₁-C₆)carbonyle, carbamyle ou alkyl(C₁-C₆)sulfonyle ; un groupement NHR" ou NR"R"' dans lesquels R" et R"', identiques ou différents, représentent un radical alkyle en C₁-C₆, un radical monohydroxyalkyle en C₁-C₆ ou un radical polyhydroxyalkyle en C₂-C₆ ;
• R₇ représente un radical alkyle en C₁-C₆ ; un radical monohydroxyalkyle en C₁-C₆ ; un radical polyhydroxyalkyle en C₂-C₆ ; un radical cyanoalkyle en C₁-C₆ ; un radical trialkyl(C₁-C₆)silanealkyle en C₁-C₆ ; un radical alcoxy(C₁-C₆)alkyle en C₁-C₆ ; un radical carbamylalkyle C₁-C₆ ; un radical alkyl(C₁-C₆)carboxyalkyle en C₁-C₆ ; un radical benzyle ;
• R₈, R₉ et R₁₀, Identiques ou différents, représentent un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical cyanoalkyle en C₁-C₆ ; un radical aryle ; un radical benzyle ; un radical amidoalkyle en C₁-C₆ ; un radical trialkyl(C₁-C₆)silanealkyle en C₁-C₆ ou un radical aminoalkyle en C₁-C₆ dont l'amine est protégée par un radical alkyl(C₁-C₆)carbonyle, amido, carboxyle, ou alkyl(C₁-C₆)sulfonyle ; deux des radicaux R₈, R₉ et R₁₀ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₈, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical cétoalkyle en C₁-C₆, un radical thio, un radical thioalkyle en C₁-C₆, un radical alkyl(C₁-C₆)thio, un radical amino, un radical amino protégé par un radical-alkyl(C₁-C₆)carbonyle, carbamyle ou alkyl(C₁-C₆)sulfonyle ;
• R₁₁ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆ ; un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• a et y sont des nombres entiers égaux à 0 ou 1 ; avec les conditions suivantes :
- dans les groupements cationiques Insaturés de formule (II) :
- lorsque a = 0, le bras de liaison D est rattaché à l'atome d'azote,
- lorsque a = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
- dans les groupements canoniques insaturés de formule (III) :
- lorsque a = 0, le bras de liaison D est rattaché à l'atome d'azote,
- lorsque a = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
- dans les groupements cationiques de formule (IV) :
- lorsque a = 0, alors le bras de liaison D est rattaché à l'atome d'azote portant les radicaux R₈ à R₁₀,
- lorsque a = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé ;
• X représente un anion monovalent ou divalent ;
étant entendu que le nombre de groupement cationique Z est au moins égal à 1.

2. Composés selon l'une quelconque des revendications précédentes, **caractérisés par le fait que** deux des radicaux R₈, R₉ et R₁₀ forment un cycle pyrrolidine, un cycle pipéridine, un cycle pipérazine ou un cycle morpholine.

3. Composés selon l'une quelconque des revendications précédentes, **caractérisés par le fait que** X ⁻ est choisi parmi un atome d'halogène, un hydroxyde, un hydrogènesulfate, ou un alkyl(C₁-C₆)sulfate.

4. Composés selon l'une quelconque des revendications précédentes, **caractérisés par le fait qu'**ils sont choisis parmi :
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le chlorure de 3-[(3-amino-pyrazolo[1,5-a]pyrimidin-7-ylcarbamoyl)-méthyl]-1-méthyl-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le méthyl sulfate de 2-[(3-amino-pyrazolo[1,5-a]pyrimidin-7-ylamino)-méthyl]-1,3-diméthyl-3H-imidazol-1-ium,
- le méthyl sulfate de 3-[(3-amino-pyrazolo[1,5-a]pyrimidin-7-ylamino)-méthyl]-1-méthyl-pyridinium,
- le méthyl sulfate de 3-[(3-amino-pyrazolo[1,5-a]pyrimidin-7-ylamino)-méthyl]-1-méthyl-pyridinium,
- le méthyl sulfate de 2-(3,7-diamino-5-méthyl-pyrazolo[1,5-a]pyrimldin-6-ylméthyl)-1,3-diméthyl-3H-imidazol-1-ium,
- le méthyl sulfate de 2-(3-amino-7-hydroxy-5-méthyl-pyrazolo[1,5-a]pyrimidin-6-ylméthyl)-1,3-diméthyl-3H-imidazol-1-ium,
- le méthyl sulfate de 2-(3,7-diamino-pyrazolo[1,5-a]pyrimidin-2-yl)-1-méthyl-pyridinium,
- le chlorure de [3-(3-amino-5-méthyl-pyrazolo[1,5-a]pyrimidin-7-ylamino)-propyl]-triméthyl-ammonium,
- le méthyl sulfate de [3-(3-amino-5-méthyl-pyrazolo[1,5-a]pyrimidin-7-ylamino)-propyl]-triméthyl-ammonium,
- le chlorure de 1-[3-(3-amino-5-méthyl-pyrazolo[1,5-a]pyrimidin-7-ylamino)-propyl]-1-méthyl-pipéridinium,
- le méthyl sulfate de 1-[3-(3-amino-5-méthyl-pyrazolo[1,5-a]pyrimidin-7-ylamino)-propyl]-1-méthyl-pipéridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition avec un acide.

5. Composés selon la revendication 4, **caractérisés par le fait qu'**ils sont choisis parmi :
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le chlorure de 3-(3-amino-7-hydroxy-5-méthyl-pyrazolo[1,5-a]pyrimidin-6-ylméthyl)-1-méthyl-pyridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition avec un acide.

6. Composés selon rune quelconque des revendications précédentes, **caractérisés par le fait que** les sels d'addition avec un acide sont choisis parmi les chlorhydrates, les bromhydrates, les sulfates, les citrates, les succinates, les tartrates, les lactates et les acétates.

7. Utilisation des composés de formule (I) tels que définis à l'une quelconque des revendications précédentes, à titre de base d'oxydation pour la teinture d'oxydation des fibres kératiniques.

8. Composition pour la teinture d'oxydation des fibres kératiniques, **caractérisée par le fait qu'**elle comprend à titre de base d'oxydation, dans un milieu approprié pour la teinture, au moins un composé de formule (i) tel que défini à l'une quelconque des revendications 1 à 6.

9. Composition selon la revendication 8, **caractérisée par le fait que** le ou les composés de formule (I) représentent de 0,0005 à 12 % en poids du poids total de la composition tinctoriale.

10. Composition selon la revendication 9, **caractérisée par le fait que** le ou les composés de formule (I) représentent de 0,005 à 6 % en poids du poids total de la composition tinctoriale.

11. Composition selon l'une quelconque des revendications 8 à 10, **caractérisée par le fait qu'**elle renferme au moins une base d'oxydation additionnelle choisie parmi les paraphénylènediamines, les bis-phénylalkylènediamines, les para-aminophénols, les ortho-aminophénols et les bases hétérocycliques différentes des composés de formule (I).

12. Composition selon la revendication 11, **caractérisée par le fait que** la ou les bases d'oxydation additionnelles représentent de 0,0005 à 12 % en poids du poids total de la composition tinctoriale.

13. Composition selon l'une quelconque des revendications 8 à 12 **caractérisée par le fait qu'**elle renferme au moins un coupleur et/ou au moins un colorant direct.

14. Composition selon la revendication 13, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

15. Composition selon la revendication 14, **caractérisée par le fait que** le ou les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

16. Composition selon l'une quelconque des revendications 13 à 15, **caractérisée par le fait que** le ou les coupleurs représentent de 0,0001 à 10 % en poids du poids total de la composition tinctoriale.

17. Composition selon l'une quelconque des revendications 8 à 17, **caractérisée par le fait que** les sels d'addition avec un acide sont choisis parmi les chlorhydrates, les bromhydrates, les sulfates, les citrates, les succinates, les tartrates, les lactates et les acétates.

18. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 8 à 17, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

19. Procédé selon la revendication 18, **caractérisé par le fait que** l'agent oxydant est choisi parmi le peroxyde d'hydrogène, le peroxyde d'urée, les bromates de métaux alcalins, les persels et les enzymes.

20. Dispositif à plusieurs compartiments, ou "kit" de teinture à plusieurs compartiments, dont un premier compartiment renferme une composition tinctoriale telle que définie à l'une quelconque des revendications 8 à 17 et un second compartiment renferme une composition oxydante.

## Claims

1. Compounds of formula (I) below, and their addition salts with an acid: 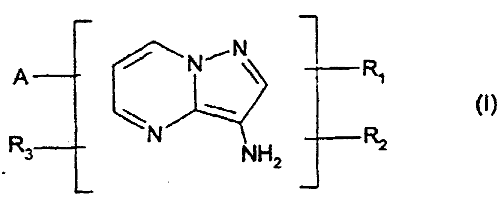 in which:
• R₁, R₂ and R₃, which may be identical or different, represent a hydrogen atom; a halogen atom; a group Z; a (C₁-C₆ alkyl)carbonyl radical; an amino(C₁-C₆ alkyl)carbonyl radical; an N-(C₁-C₆ alkyl)amino(C₁-C₆ alkyl)carbonyl radical; an N,N-di(C₁-C₆ alkyl)amino(C₁-C₆ alkyl)carbonyl radical; an amino(C₁-C₆ alkyl)carbonyl(C₁-C₆ alkyl) radical; an N-(C₁-C₆ alkyl)amino (C₁-C₆ alkyl)carbonyl(C₁-C₆ alkyl) radical; an N,N-di(C₁-C₆ alkyl)amino(C₁-C₆ alkyl)carbonyl(C₁-C₆ alkyl) radical; a carboxyl radical; a (C₁-C₆ alkyl)carboxyl radical; a (C₁-C₆ alkyl)sulphonyl radical; an aminosulphonyl radical; an N-(C₁-C₆ alkyl)aminosulphonyl radical; an N,N-di(C₁-C₆ alkyl)aminosulphonyl radical; an aminosulphonyl(C₁-C₆ alkyl) radical; an N-(C₁-C₆ alkyl)aminosulphonyl(C₁-C₆ alkyl) radical; an N,N-di(C₁-C₆ alkyl)aminosulphonyl(C₁-C₆ alkyl) radical; a carbamyl radical; an N-(C₁-C₆ alkyl)carbamyl radical; an N,N-di(C₁-C₆ alkyl)carbamyl radical; a carbamyl(C₁-C₆ alkyl) radical; an N-(C₁-C₆ alkyl)carbamyl(C₁-C₆ alkyl) radical; an N,N-di(C₁-C₆ alkyl)carbamyl(C₁-C₆ alkyl) radical; a C₁-C₆ alkyl radical; a hydroxyl radical; a nitro radical; a C₁-C₆ monohydroxyalkyl radical; a C₂-C₆ polyhydroxyalkyl radical; a C₁-C₆ (C₁-C₆ alkoxy)alkyl radical; a C₁-C₆ trifluoroalkyl radical; a cyano radical; a group OR₆ or SR₆; an amino radical; an N-(C₁-C₆ alkyl)amino radical; an N,N-di(C₁-C₆ alkyl)amino radical (where the two alkyl substituents may form a 5- or 6-membered ring); an N-hydroxy(C₁-C₆ alkyl)amino radical; an N,N-bis(hydroxy(C₁-C₆ alkyl))amino radical; an N-polyhydroxy(C₂-C₆ alkyl)amino radical; an N,N-bis(polyhydroxy(C₂-C₆ alkyl))amino radical; an amino(C₁-C₆ alkyl)amino radical in which the terminal amino group is unsubstituted or substituted by one or two C₁-C₆ alkyl radicals, where the said alkyl radicals may form a saturated or unsaturated, 5- or 6-membered ring; an amino group protected by a (C₁-C₆ alkyl)carbonyl, trifluoro(C₁-C₆ alkyl)carbonyl, amino(C₁-C₆ alkyl)carbonyl, N-Z-amino(C₁-C₆ alkyl)-carbonyl, N-(C₁-C₆ alkyl)amino(C₁-C₆ alkyl)carbonyl, N,N-di(C₁-C₆ alkyl)amino(C₁-C₆ alkyl)carbonyl or formyl radical or by a group Z ;
• R₆ denotes a C₁-C₆ alkyl radical; a C₁-C₆ monohydroxyalkyl radical; a C₂-C₆ polyhydroxyalkyl radical; a group Z; a C₁-C₆ (C₁-C₆ alkoxy)alkyl radical; an aryl radical; a benzyl radical; a C₁-C₆ carboxyalkyl radical; a C₁-C₆ (C₁-C₆ alkyl)carboxyalkyl radical; a C₁-C₆ cyanoalkyl radical; a C₁-C₆ carbamylalkyl radical; a C₁-C₆ N-(C₁-C₆ alkyl)carbamylalkyl radical; a C₁-C₆ N,N-di(C₁-C₆ alkyl)carbamylalkyl radical; a C₁-C₆ trifluoroalkyl radical; a C₁-C₆ aminosulphonylalkyl radical; a C₁-C₆ N-(C₁-C₆ alkyl)aminosulphonylalkyl radical; a C₁-C₆ N,N-di(C₁-C₆ alkyl)aminosulphonylalkyl radical; a C₁-C₆ (C₁-C₆ alkyl)sulphinylalkyl radical; a C₁-C₆ (C₁-C₆ alkyl)sulphonylalkyl radical; a C₁-C₆ (C₁-C₆ alkyl)carbonylalkyl radical; a C₁-C₆ aminoalkyl radical; a C₁-C₆ aminoalkyl radical whose amine is substituted by one or two identical or different radicals selected from C₁-C₆ alkyl, monohydroxy(C₁-C₆ alkyl), polyhydroxy(C₂-C₆ alkyl), (C₁-C₆ alkyl)-carbonyl, formyl, trifluoro(C₁-C₆ alkyl)carbonyl and (C₁-C₆ alkyl)sulphonyl radicals or by a group Z;
• A represents a group -NR₄R₅ or a hydroxyl radical;
• R₄ and R₅, which are identical or different, represent a hydrogen atom; a group Z; a C₁-C₆ alkyl radical; a C₁-C₆ monohydroxyalkyl radical; a C₂-C₆ polyhydroxyalkyl radical; a C₁-C₆ (C₁-C₆ alkoxy)alkyl radical; an aryl radical; a benzyl radical; a C₁-C₆ cyanoalkyl radical; a C₁-C₆ carbamylalkyl radical; a C₁-C₆ N-(C₁-C₆ alkyl)carbamylalkyl radical; a C₁-C₆ N,N-di(C₁-C₆ alkyl)carbamylalkyl radical; a C₁-C₆ thiocarbamylalkyl radical; a C₁-C₆ trifluoroalkyl radical; a C₁-C₆ sulphoalkyl radical; a C₁-C₆ (C₁-C₆ alkyl)carboxyalkyl radical; a C₁-C₆ (C₁-C₆ alkyl)sulphinylalkyl radical; a C₁-C₆ aminosulphonylalkyl radical; a C₁-C₆ N-(C₁-C₆ alkyl)-aminosulphonylalkyl radical; a C₁-C₆ N,N-di(C₁-C₆ alkyl)aminosulphonylalkyl radical; a C₁-C₆ (C₁-C₆ alkyl)carbonylalkyl radical; a C₁-C₆ aminoalkyl radical; a C₁-C₆ aminoalkyl radical whose amine is substituted by one or two identical or different radicals selected from C₁-C₆ alkyl, C₁-C₆ monohydroxyalkyl, C₂-C₆ polyhydroxyalkyl, (C₁-C₆ alkyl)carbonyl, (C₁-C₆ alkyl)sulphonyl, formyl and trifluoro(C₁-C₆ alkyl)carbonyl radicals or by a group Z;
one and only one of the radicals R₄ and R₅ may also represent a (C₁-C₆ alkyl)carbonyl; formyl; trifluoro(C₁-C₆ alkyl)carbonyl; amino(C₁-C₆ alkyl)carbonyl; N-(C₁-C₆ alkyl)amino(C₁-C₆ alkyl)carbonyl; or N,N-di(C₁-C₆ alkyl)amino(C₁-C₆ alkyl)carbonyl radical;
• Z is selected from the unsaturated cationic groups of formulae (II) and (III) below and the saturated cationic groups of formula (IV) below: 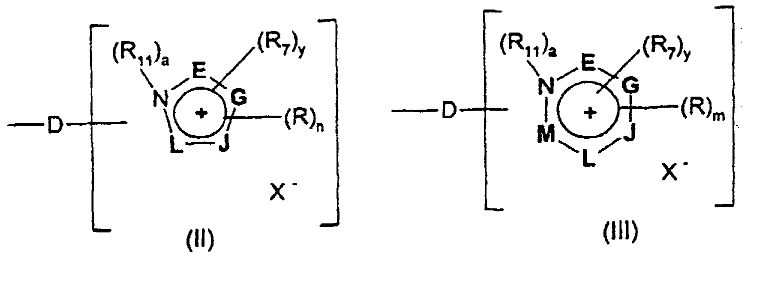 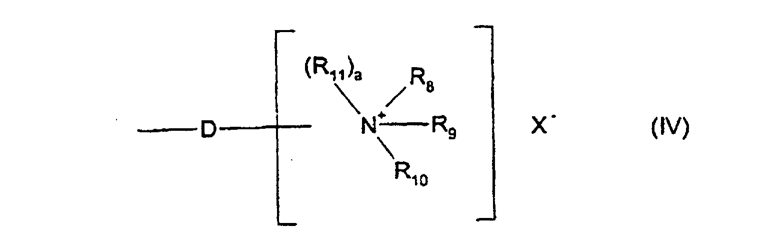
in which:
• D is a linker which represents an alkyl chain containing preferably 1 to 14 carbon atoms, is linear or branched and may be interrupted by one or more heteroatoms such as oxygen, sulphur or nitrogen atoms and may be substituted by one or more hydroxyl or C₁-C₆ alkoxy radicals, and may carry one or more ketone functions;
• the ring members E, G, J, L and M, which are identical or different, form rings, which are selected from pyrrole, imidazole, pyrazole, oxazole, thiazole, triazole, pyridine, pyrimidine, pyrazine, oxazine and triazine rings, with the nitrogen of the ring;
• n is an integer between 0 and 4, inclusively;
• m is an integer between 0 and 5, inclusively;
• the radicals R, which are identical or different, represent a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a C₁-C₆ tri(C₁-C₆ alkyl)silanealkyl radical, an amido radical, an aldehydo radical, a carboxyl radical, a C₁-C₆ alkylcarbonyl radical, a thio radical, a C₁-C₆ thioalkyl radical, a (C₁-C₆ alkyl)thio radical, an amino radical, an amino radical protected by a (C₁-C₆ alkyl)carbonyl, carbamyl or (C₁-C₆ alkyl)sulphonyl radical; a group NHR" or NR"R in which R" and R , which are identical or different, represent a C₁-C₆ alkyl radical, a C₁-C₆ monohydroxyalkyl radical or a C₂-C₆ polyhydroxyalkyl radical;
• R₇ represents a C₁-C₆ alkyl radical, a C₁-C₆ monohydroxyalkyl radical, a C₂-C₆ polyhydroxyalkyl radical, a C₁-C₆ cyanoalkyl radical, a C₁-C₆ tri(C₁-C₆ alkyl)silanealkyl radical, a C₁-C₆ (C₁-C₆ alkoxy)alkyl radical, a carbamyl(C₁-C₆ alkyl) radical, a C₁-C₆ (C₁-C₆ alkyl)carboxyalkyl radical, a benzyl radical;
• R₈, R₉ and R₁₀, which are identical or different, represent a C₁-C₆ alkyl radical, a C₁-C₆ monohydroxyalkyl radical, a C₂-C₆ polyhydroxyalkyl radical, a C₁-C₆ (C₁-C₆ alkoxy)alkyl radical, a C₁-C₆ cyanoalkyl radical, an aryl radical, a benzyl radical, a C₁-C₆ amidoalkyl radical, a C₁-C₆ tri(C₁-C₆ alkyl)silanealkyl radical or a C₁-C₆ aminoalkyl radical whose amine is protected by a (C₁-C₆ alkyl)carbonyl, amido, carboxyl or (C₁-C₆ alkyl)sulphonyl radical; two of the radicals R₈, R₉ and R₁₀ may also form, together with the nitrogen atom to which they are attached, a saturated 5- or 6-membered carbon-containing ring or one containing one or more heteroatoms, it being possible for the said ring to be unsubstituted or substituted by a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a C₁-C₆ tri(C₁-C₆ alkyl)silanealkyl radical, an amido radical, an aldehydo radical, a carboxyl radical, a C₁-C₆ ketoalkyl radical, a thio radical, a C₁-C₆ thioalkyl radical, a (C₁-C₆ alkyl)thio radical, an amino radical, or an amino radical protected by a (C₁-C₆ alkyl)carbonyl, carbamyl or (C₁-C₆ alkyl)sulphonyl radical;
• R₁₁ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical; a C₁-C₆ aminoalkyl radical whose amine is protected by a (C₁-C₆ alkyl)carbonyl, carbamyl or (C₁-C₆ alkyl)sulphonyl radical; a C₁-C₆ carboxyalkyl radical; a C₁-C₆ cyanoalkyl radical; a C₁-C₆ carbamylalkyl radical; a C₁-C₆ trifluoroalkyl radical; a C₁-C₆ tri(C₁-C₆ alkyl)silanealkyl radical; a C₁-C₆ sulphonamidoalkyl radical; a C₁-C₆ (C₁-C₆ alkyl)carboxyalkyl radical; a C₁-C₆ (C₁-C₆ alkyl)-sulphinylalkyl radical; a C₁-C₆ (C₁-C₆ alkyl)sulphonylalkyl radical; a C₁-C₆ (C₁-C₆ alkyl)ketoalkyl radical; a C₁-C₆ N-(C₁-C₆ alkyl)carbamylalkyl radical; a C₁-C₆ N-(C₁-C₆ alkyl)sulphonamidoalkyl radical;
• a and y are integers equal to 0 or 1; with the following conditions:
- in the unsaturated cationic groups of formula (II) :
- when a = 0, the linker D is attached to the nitrogen atom,
- when a = 1, the linker D is attached to one of the ring members E, G, J or L,
- y can adopt the value 1 only
1) when the ring members E, G, J and L simultaneously represent a carbon atom and when the radical R₇ is carried by the nitrogen atom of the unsaturated ring; or else
2) when at least one of the ring members E, G, J and L represents a nitrogen atom to which the radical R₇ is attached;
- in the unsaturated cationic groups of formula (III) :
- when a = 0, the linker D is attached to the nitrogen atom,
- when a = 1, the linker D is attached to one of the ring members E, G, J, L or M,
- y can adopt the value 1 only when at least one of the ring members E, G, J, L and M represents a divalent atom and when the radical R₇ is carried by the nitrogen atom of the unsaturated ring;
- in the cationic groups of formula (IV):
- when a = 0, then the linker D is attached to the nitrogen atom which carries the radicals R₈ to R₁₀,
- when a = 1, then two of the radicals R₈ to R₁₀, together with the nitrogen atom to which they are attached, form a 5- or 6-membered saturated ring as defined above, and the linker D is carried by a carbon atom of the said saturated ring;
• X⁻ represents a monovalent or divalent anion;
with the proviso that the number of cationic groups Z is at least 1.

2. Compounds according to any one of the preceding claims, **characterized in that** two of the radicals R₈, R₉ and R₁₀ form a pyrrolidine ring, a piperidine ring, a piperazine ring or a morpholine ring.

3. Compounds according to any one of the preceding claims, **characterized in that** X⁻ represents a halogen atom, a hydroxide, a hydrogen sulphate or a C₁-C₆ alkyl sulphate.

4. Compounds according to any one of the preceding claims, **characterized in that** they are selected from:
- 3-[3-(3-amino-5-methylpyrazolo[1,5-a]pyrimidin-7-ylamino)propyl]-1-(2-hydroxyethyl)-3H-imidazol-1-ium chloride,
- 3-[(3-aminopyrazolo[1,5-a]pyrimidin-7-ylcarbamoyl)-methyl]-1-methyl-3H-imidazol-1-ium chloride,
- 3-(3-amino-7-hydroxy-5-methylpyrazolo[1,5-a]-pyrimidin-6-ylmethyl)-1-methylpyridinium methyl sulphate,
- 3-(3-amino-7-hydroxy-5-methylpyrazolo[1,5-a]pyrimidin-6-ylmethyl)-1-(2-hydroxyethyl)pyridinium chloride,
- 2-[(3-aminopyrazolo[1,5-a]pyrimidin-7-ylamino)methyl]-1,3-dimethyl-3H-imidazol-1-ium methyl sulphate,
- 3-[(3-aminopyrazolo[1,5-a]pyrimidin-7-ylamino)methyl]-1-methylpyridinium methyl sulphate,
- 3-[(3-aminopyrazolo[1,5-a]pyrimidin-7-ylamino)methyl]-1-methylpyridinium methyl sulphate,
- 2-(3,7-diamino-5-methylpyrazolo[1,5-a]pyrimidin-6-ylmethyl)-1,3-dimethyl-3H-imidazol-1-ium methyl sulphate,
- 2-(3-amino-7-hydroxy-5-methylpyrazolo[1,5-a]pyrimidin-6-ylmethyl)-1,3-dimethyl-3H-imidazol-1-ium methyl sulphate,
- 2-(3,7-diaminopyrazolo[1,5-a]pyrimidin-2-yl)-1-methylpyridinium methyl sulphate,
- [3-(3-amino-5-methylpyrazolo[1,5-a]pyrimidin-7-ylamino)propyl]trimethylammonium chloride,
- [3-(3-amino-5-methylpyrazolo[1,5-a]pyrimidin-7-ylamino)propyl]trimethylammonium methyl sulphate,
- 1-[3-(3-amino-5-methylpyrazolo[1,5-a]pyrimidin-7-ylamino)propyl]-1-methylpiperidinium chloride,
- 1-[3-(3-amino-5-methylpyrazolo[1,5-a]pyrimidin-7-ylamino)propyl]-1-methylpiperidinium methyl sulphate,
- 4-[3-(3-amino-5-methylpyrazolo[1,5-a]pyrimidin-7-ylamino)propyl]-4-methylmorpholin-4-ium chloride,
- 4-[3-(3-amino-5-methylpyrazolo[1,5-a]pyrimidin-7-ylamino)propyl]-4-methylmorpholin-4-ium methyl sulphate,
and their addition salts with an acid.

5. Compounds according to Claim 4, **characterized in that** they are selected from:
- 3-[3-(3-amino-5-methylpyrazolo[1,5-a]pyrimidin-7-ylamino)propyl]-1-(2-hydroxyethyl)-3H-imidazol-1-ium chloride,
- 3-(3-amino-7-hydroxy-5-methylpyrazolo[1,5-a]-pyrimidin-6-ylmethyl)-1-methylpyridinium methyl sulphate,
- 3-(3-amino-7-hydroxy-5-methylpyrazolo[1,5-a]-pyrimidin-6-ylmethyl)-1-(2-hydroxyethyl)pyridinium chloride,
- 3-(3-amino-7-hydroxy-5-methylpyrazolo[1,5-a]-pyrimidin-6-ylmethyl)-1-methylpyridinium chloride,
- 4-[3-(3-amino-5-methylpyrazolo[1,5-a]pyrimidin-7-ylamino)propyl]-4-methylmorpholin-4-ium chloride,
- 4-[3-(3-amino-5-methylpyrazolo[1,5-a]pyrimidin-7-ylamino)propyl]-4-methylmorpholin-4-ium methyl sulphate,
and their addition salts with an acid.

6. Compounds according to any one of the preceding claims, **characterized in that** the addition salts with an acid are selected from the hydrochlorides, hydrobromides, sulphates, citrates, succinates, tartrates, lactates and acetates.

7. Use of the compounds of formula (I) as defined in any one of the preceding claims as oxidation base for the oxidation dyeing of keratinous fibres.

8. Composition for the oxidation dyeing of keratinous fibres, **characterized in that** it comprises, as oxidation base, in a medium appropriate for dyeing, at least one compound of formula (I) as defined in any one of Claims 1 to 6.

9. Composition according to Claim 8, **characterized in that** the compound or compounds of formula (I) represent(s) from 0.0005 to 12% by weight of the total weight of the dyeing composition.

10. Composition according to Claim 9, **characterized in that** the compound or compounds of formula (I) represent(s) from 0.005 to 6% by weight of the total weight of the dyeing composition.

11. Composition according to any one of Claims 8 to 10, **characterized in that** it includes at least one additional oxidation base selected from para-phenylenediamines, bisphenylalkylenediamines, para-aminophenols, ortho-aminophenols and heterocyclic bases other than the compounds of formula (I).

12. Composition according to Claim 11, **characterized in that** the additional oxidation base or bases represent(s) from 0.0005 to 12% by weight of the total weight of the dyeing composition.

13. Composition according to any one of Claims 8 to 12, **characterized in that** it includes at least one coupler and/or at least one direct dye.

14. Composition according to Claim 13, **characterized in that** the coupler or couplers is or are selected from meta-phenylenediamines, meta-aminophenols, meta-diphenols and heterocyclic couplers, and their addition salts with an acid.

15. Composition according to Claim 14, **characterized in that** the coupler or couplers is or are selected from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one, 1-phenyl-3-methylpyrazol-5-one, and their addition salts with an acid.

16. Composition according to any one of Claims 13 to 15, **characterized in that** the coupler or couplers represent(s) from 0.0001 to 10% by weight of the total weight of the dyeing composition.

17. Composition according to any one of Claims 8 to 17, **characterized in that** the addition salts with an acid are selected from the hydrochlorides, hydrobromides, sulphates, citrates, succinates, tartrates, lactates and acetates.

18. Method of dyeing keratinous fibres and, in particular, human keratinous fibres such as the hair, **characterized in that** at least one dyeing composition as defined in any one of Claims 8 to 17 is applied to these fibres, and **in that** the colour is revealed at an acidic, neutral or alkaline pH with the aid of an oxidizing agent which is added to the dyeing composition right at the time of use or which is present in an oxidizing composition which is applied simultaneously or sequentially, separately.

19. Method according to Claim 18, **characterized in that** the oxidizing agent is selected from hydrogen peroxide, urea peroxide, alkali metal bromates, persalts and enzymes.

20. Multi-compartment device or multi-compartment dyeing kit of which a first compartment contains a dyeing composition as defined in any one of Claims 8 to 17 and a second compartment contains an oxidizing composition.

## Patentansprüche

1. Verbindungen der folgenden Formel (I) und ihre Additionssalze mit einer Säure: 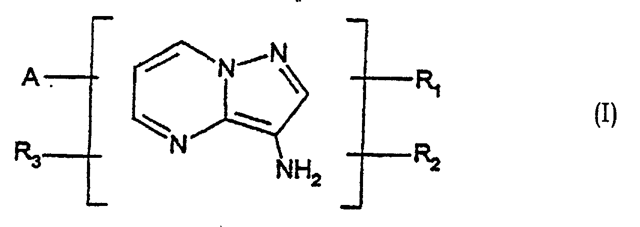 worin bedeuten:
R₁, R₂ und R₃, die identisch oder voneinander verschieden sein können, Wasserstoff; Halogen; eine Gruppe Z; Alkyl(C₁₋₆)carbonyl; Aminoalkyl(C₁₋₆)carbonyl; N-Alkyl(C₁₋₆)-aminoalkyl(C₁₋₆)carbonyl; N,N-Dialkyl(C₁₋₆)aminoalkyl(C₁₋₆)carbonyl; Aminoalkyl(C₁₋₆)carbonylalkyl(C₁₋₆); N-Alkyl(C₁₋₆)aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Alkyl(C₁₋₆)-aminosulfonyl; N,N-Dialkyl(C₁₋₆)aminosulfonyl; Aminosulfonylalkyl(C₁₋₆); N-Alkyl(C₁₋₆)aminosulfonylalkyl(C₁₋₆); N,N-Dialkyl(C₁₋₆)-aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); C₁₋₆-Alkyl; Hydroxy; Nitro; C₁₋₆-Monohydroxyalkyl; C₂₋₆-Polyhydroxyalkyl; Alkoxy(C₁₋₆)alkyl(C₁₋₆); C₁₋₆-Trifluoralkyl; Cyano; OR₆ oder SR₆; Amino; N-Alkyl(C₁₋₆)amino; N,N-Dialkyl(C₁₋₆)amino (wobei die beiden Substituenten an der Alkylgruppe einen 5- oder 6-gliedrigen Ring bilden können); N-Hydroxyalkyl(C₁₋₆)amino; N,N-Bis(hydroxyalkyl(C₁₋₆))amino; N-Polyhydroxyalkyl(C₂₋₆)amino; N,N-Bis(polyhydroxyalkyl(C₂₋₆))amino; Aminoalkyl(C₁₋₆)amino, wobei die endständige Aminogruppe unsubstituiert vorliegt oder mit einer oder zwei C₁₋₆-Alkylguppen substituiert ist, wobei die Alkylgruppen einen gesättigten oder ungesättigten 5- oder 6-gliedrigen Ring bilden können; eine Aminogruppe, die mit einer der folgenden Gruppen geschützt ist: Alkyl(C₁₋₆)carbonyl, Trifluoralkyl(C₁₋₆)-carbonyl, Aminoalkyl(C₁₋₆)carbonyl, N-Z-Aminoalkyl(C₁₋₆)carbonyl, N-Alkyl(C₁₋₆)-aminoalkyl(C₁₋₆)-carbonyl, N,N-Dialkyl(C₁₋₆)-aminoalkyl(C₁₋₆)-carbonyl, Formyl oder einer Gruppe Z;
R₆ C₁₋₆-Alkyl; C₁₋₆-Monohydroxyalkyl; C₂₋₆-Polyhydroxyalkyl; eine Gruppe Z; Alkoxy(C₁₋₆)alkyl(C₁₋₆); Aryl; Benzyl; C₁₋₆-Carboxyalkyl; Alkyl(C₁₋₆)carboxyalkyl(C₁₋₆); C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Trifluoralkyl; C₁₋₆-Aminosulfonylalkyl; N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); C₁₋ₑ-Aminoalkyl; C₁₋₆-Aminoalkyl, wobei die Aminogruppe mit einer oder zwei Gruppen substituiert ist, die gleich oder verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)carbonyl, Alkyl(C₁₋₆)sulfonyl oder einer Gruppe Z;
A eine Gruppe -NR₄R₅ oder eine Hydroxygruppe;
R₄ und R₅, die identisch oder voneinander verschieden sind, Wasserstoff; eine Gruppe Z; C₁₋₆-Alkyl; C₁₋₆-Monohydroxyalkyl; C₂₋₆-Polyhydroxyalkyl; Alkoxy(C₁₋₆)alkyl(C₁₋₆); Aryl; Benzyl; C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)carbamoylalkyl(C₁₋₆); C₁₋₆-Thiocarbamoylalkyl; C₁₋₆-Trifluoralkyl; C₁₋₆-Sulfoalkyl; Alkyl(C₁₋₆)carboxyalkyl(C₁₋₆); Alkyl(C₁₋₆)sulfinylalkyl(C₁₋₆); C₁₋₆-Aminosulfonylalkyl; N-Alkyl(C₁₋₆)-aminosulfonylalkyl(C₁₋₆); N,N-Dialkyl(C₁₋₆)aminosulfonylalkyl(C₁₋₆); Alkyl(C₁₋₆)carbonylalkyl(C₁₋₆); C₁₋₆-Aminoalkyl; C₁₋₆-Aminoalkyl, wobei die Aminogruppe mit einer oder zwei Gruppen substituiert ist, die gleich oder verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Alkyl-(C₁₋₆)sulfonyl, Formyl, Trifluoralkyl(C₁₋₆)carbonyl oder einer Gruppe Z;
wobei eine der Gruppen R₄ und R₅, jedoch lediglich eine Gruppe, auch eine Gruppe Alkyl(C₁₋₆)carbonyl, Formyl, Trifluoralkyl(C₁₋₆)-carbonyl, Aminoalkyl(C₁₋₆)carbonyl, N-Alkyl(C₁₋₆)aminoalkyl(C₁₋₆)-carbonyl; N,N-Dialkyl(C₁₋₆)aminoalkyl(C₁₋₆)carbonyl bedeuten kann;
Z ist unter den ungesättigten kationischen Gruppen der folgenden Formeln (II) und (III) und den gesättigten kationischen Gruppen der folgenden Formel (IV) ausgewählt: 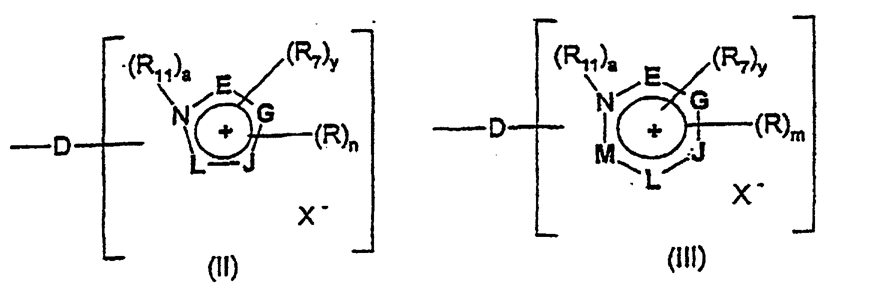 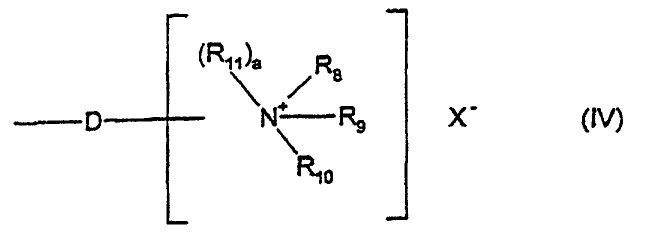
worin:
- D eine Verbindungsgruppe ist, die eine Alkylkette mit vorzugsweise 1 bis 14 Kohlenstoffatomen bedeutet, die geradkettig oder verzweigt vorliegt, mit einem oder mehreren Heteroatomen, wie beispielsweise Sauerstoff, Schwefel oder Stickstoff, unterbrochen sein kann, mit einer oder mehreren Hydroxygruppen oder C₁₋₆-Alkoxygruppen substituiert sein kann und eine oder mehrere Ketogruppen tragen kann;
- E, G, J, L und M, die identisch oder voneinander verschieden sind, mit dem Stickstoffatom des Rings Ringe bilden, die unter den folgenden Ringen ausgewählt sind: Pyrrol, Imidazol, Pyrazol, Oxazol, Thiazol, Triazol, Pyridin, Pyrimidin, Pyrazin, Oxazin und Triazin;
- n 0 oder eine ganze Zahl von 1 bis 4 ist;
- m 0 oder eine ganze Zahl von 1 bis 5 ist;
- die Gruppen R, die identisch oder voneinander verschieden sind, Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Alkylcarbonyl, Thio, C₁₋₆-Thioalkyl, Alkyl(C₁₋₆)thio, Amino, eine Aminogruppe, die mit Alkyl(C₁₋₆)carbonyl, Carbamoyl oder Alkyl(C₁₋₆)sulfonyl geschützt ist; oder eine Gruppe NHR" oder NR"R"' bedeuten, worin R" und R''', die identisch oder voneinander verschieden sind, C₁₋₆-Alkyl, C₁₋₆-Monohydroxyalkyl oder C₂₋₆-Polyhydroxyalkyl bedeuten;
- R₇ C₁₋₆-Alkyl, C₁₋₆-Monohydroxyalkyl, C₂₋₆-Polyhydroxyalkyl, C₁₋₆-Cyanoalkyl, Trialkyl(C₁₋₆)silanalkyl(C₁₋₆), Alkoxy(C₁₋₆)-alkyl(C₁₋₆), C₁₋₆-Carbamoylalkyl, Alkyl(C₁₋₆)carboxyalkyl(C₁₋₆) oder Benzyl bedeutet;
- R₈, R₉ oder R₁₀, die gleich oder verschieden sind, bedeuten: C₁₋₆-Alkyl, C₁₋₆-Monohydroxyalkyl, C₂₋₆-Polyhydroxyalkyl, Alkoxy(C₁₋₆)alkyl(C₁₋₆), C₁₋₆-Cyanoalkyl, Aryl, Benzyl, C₁₋₆-Amidoalkyl, Trialkyl(C₁₋₆)silanalkyl(C₁₋₆) oder C₁₋₆-Aminoalkyl, wobei die Aminogruppe mit Alkyl(C₁₋₆)carbonyl, Amido, Carboxy oder Alkyl(C₁₋₆)sulfonyl geschützt ist; und zwei der Gruppen R₈, R₉ und R₁₀ auch gemeinsam mit dem Stickstoffatom, an das sie gebunden sind, einen 5- oder 6-gliedrigen gesättigten Ring bilden können, der Kohlenstoffatome enthält oder ein oder mehrere Heteroatome aufweist, wobei der Ring unsubstituiert vorliegen kann oder substituiert sein kann mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Ketoalkyl, Thio, C₁₋₆-Thioalkyl, Alkyl(C₁₋₆)thio, Amino, Amino, das mit Alkyl(C₁₋₆)carbonyl, Carbamoyl oder Alkyl(C₁₋₆) sulfonyl geschützt ist;
- R₁₁ C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)-silanalkyl(C₁₋₆); C₁₋₆-Sulfonamidoalkyl; Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
- a und y 0 oder 1 bedeuten; mit den folgenden Bedingungen:
- in den ungesättigten kationischen Gruppen der Formel (II):
- die Verbindungsgruppe D ist an das Stickstoffatom gebunden, wenn a = 0.
- die Verbindungsgruppe D ist an eines der Atome E, G, J oder L gebunden, wenn a = 1,
- y kann nur den Wert 1 annehmen, wenn:
1) die Atome E, G, J und L gleichzeitig ein Kohlenstoffatom bedeuten und die Gruppe R₇ von dem Stickstoffatom des ungesättigten Rings getragen wird; oder
2) mindestens eines der Atome E, G, J und L ein Stickstoffatom bedeutet, an das R₇ gebunden ist;
- in den ungesättigten kationischen Gruppen der Formel (III):
- die Verbindungsgruppe D an das Stickstoffatom gebunden ist, wenn a = 0,
- die Verbindungsgruppe D an eines der Atome E, G, J, L oder M gebunden ist, wenn a = 1,
- y nur den Wert 1 annehmen kann, wenn mindestens eines der Atome E, G, J, L und M ein zweiwertiges Atom bedeutet und die Gruppe R₇ von dem Stickstoffatom des ungesättigten Rings getragen wird;
- in den kationischen Gruppen der Formel (IV):
- die Verbindungsgruppe D an das Stickstoffatom gebunden ist, das die Gruppen R₈ bis R₁₀ trägt, wenn a = 0,
- zwei der Gruppen R₈ bis R₁₀ gemeinsam mit dem Stickstoffatom, an das sie gebunden sind, einen oben definierten gesättigten 5- oder 6-gliedrigen Ring bilden und die Verbindungsgruppe D von einem Kohlenstoffatom des gesättigten Rings getragen wird, wenn a = 1;
- X⁻ ein einwertiges oder zweiwertiges Anion bedeutet;
mit der Maßgabe, daß:
- die Anzahl der kationischen Gruppen Z mindestens 1 ist.

2. Verbindungen nach einem der vorhergehenden Ansprüche, **dadurch gekennzeichnet, daß** zwei der Gruppen R₈, R₉ und R₁₀ einen Pyrrolidinring, Piperidinring, Piperazinring oder Morpholinring bilden.

3. Verbindungen nach einem der vorhergehenden Ansprüche, **dadurch gekennzeichnet, daß** X⁻ ein Halogenatom, ein Hydroxid, ein Hydrogensulfat oder ein Alkyl(C₁₋₆)sulfat bedeutet.

4. Verbindungen nach einem der vorhergehenden Ansprüche, **dadurch gekennzeichnet, daß** sie ausgewählt sind unter:
- 3-[3-(3-Amino-5-methyl-pyrazolo[1,5-a]pyrimidin-7-ylamino)-propyl]-1-(2-hydroxyethyl]-3H-imidazol-1-ium-chlorid;
- 3-[(3-Amino-pyrazolo[1,5-a]pyrimidin-7-ylcarbamoyl)-methyl]-1-methyl-3H-imidazol-1-ium-chlorid;
- 3-(3-Amino-7-hydroxy-5-methyl-pyrazolo[1,5-a]pyrimidin-6-ylmethyl)-1-methyl-pyridinium-methylsulfat;
- 3-(3-Amino-7-hydroxy-5-methyl-pyrazolo[1,5-a]pyrimidin-6-ylmethyl)-1-(2-hydroxyethyl]-pyridinium-chlorid;
- 2-[(3-Amino-pyrazolo[1,5-a]pyrimidin-7-ylamino)-methyl]-1,3-dimethyl-3H-imidazol-1-ium-methylsulfat;
- 3-[(3-Amino-pyrazolo[1,5-a]pyrimidin-7-ylamino)-methyl]-1-methyl-pyridinium-methylsulfat;
- 3-[(3-Amino-pyrazolo[1,5-a]pyrimidin-7-ylamino)-methyl]-1-methyl-pyridinium-methylsulfat;
- 2-(3,7-Diamino-5-methyl-pyrazolo[1,5-a]pyrimidin-6-ylmethyl)-1,3-dimethyl-3H-imidazol-1-ium-methylsulfat;
- 2-(3-Amino-7-hydroxy-5-methyl-pyrazolo[1,5-a]pyrimidin-6-ylmethyl)-1,3-dimethyl-3H-imidazol-1-ium-methylsulfat;
- 2-(3,7-Diamino-pyrazolo[1,5-a]pyrimidin-2-yl)-1-methylpyridinium-methylsulfat;
- [3-(3-Amino-5-methyl-pyrazolo[1,5-a]pyrimidin-7-ylamino)-propyl]-trimethyl-ammonium-chlorid;
- [3-(3-Amino-5-methyl-pyrazolo[1,5-a]pyrimidin-7-ylamino)-propyl]-trimethyl-ammonium-methylsulfat;
- 1-[3-(3-Amino-5-methyl-pyrazolo[1,5-a]pyrimidin-7-ylamino)-propyl]-1-methyl-piperidinium-chlorid;
- 1-[3-(3-Amino-5-methyl-pyrazolo[1,5-a]pyrimidin-7-ylamino)-propyl]-1-methyl-piperidinium-methylsulfat;
- 4-[3-(3-Amino-5-methyl-pyrazolo[1,5-a]pyrimidin-7-ylamino)-propyl]-4-methyl-morpholin-4-ium-chlorid;
- 4-[3-(3-Amino-5-methyl-pyrazolo[1,5-a]pyrimidin-7-ylamino)-propyl]-4-methyl-morpholin-4-ium-methylsulfat;
- und ihren Additionssalzen mit einer Säure.

5. Verbindungen nach Anspruch 4, **dadurch gekennzeichnet, daß** sie ausgewählt sind unter:
- 3-[3-(3-Amino-5-methyl-pyrazolo[1,5-a]pyrimidin-7-ylamino)-propyl]-1-(2-hydroxyethyl]-3H-imidazol-1-ium-chlorid;
- 3-(3-Amino-7-hydroxy-5-methyl-pyrazolo[1,5-a]pyrimidin-6-ylmethyl)-1-methyl-pyridinium-methylsulfat;
- 3-(3-Amino-7-hydroxy-5-methyl-pyrazolo[1,5-a]pyrimidin-6-ylmethyl)-1-(2-hydroxyethyl]-pyridinium-chlorid;
- 3-(3-Amino-7-hydroxy-5-methyl-pyrazolo[1,5-a]pyrimidin-6-ylmethyl)-1-methyl-pyridinium-chlorid;
- 4-[3-(3-Amino-5-methyl-pyrazolo[1,5-a]pyrimidin-7-ylamino)-propyl]-4-methyl-morpholin-4-ium-chlorid;
- 4-[3-(3-Amino-5-methyl-pyrazolo[1,5-a]pyrimidin-7-ylamino)-propyl]-4-methyl-morpholin-4-ium-methylsulfat;
- und ihren Additionssalzen mit einer Säure.

6. Verbindungen nach einem der vorhergehenden Ansprüche, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

7. Verwendung von Verbindungen der Formel (I) nach einem der vorhergehenden Ansprüche als Oxidationsbasen zum oxidativen Färben von Keratinfasern.

8. Zusammensetzung zum oxidativen Färben von Keratinfasern, **dadurch gekennzeichnet, daß** sie als Oxidationsbase in einem zum Färben geeigneten Medium mindestens eine Verbindung der Formel (I) nach einem der Ansprüche 1 bis 6 enthält.

9. Zusammensetzung nach Anspruch 8, **dadurch gekennzeichnet, daß** die Verbindung(en) der Formel (I) 0,0005 bis 12 Gew.-% des Gesamtgewichts der Farbmittelzusammensetzung ausmachen.

10. Zusammensetzung nach Anspruch 9, **dadurch gekennzeichnet, daß** die Verbindung(en) der Formel (I) 0,005 bis 6 Gew.-% des Gesamtgewichts der Farbmittelzusammensetzung ausmachen.

11. Zusammensetzung nach einem der Ansprüche 8 bis 10, **dadurch gekennzeichnet, daß** sie mindestens eine ergänzende Oxidationsbase enthält, die unter den p-Phenylendiaminen, Bisphenylalkylendiaminen, p-Aminophenolen, o-Aminophenolen und heterocyclischen Basen, die von den Verbindungen der Formel (I) verschieden sind, ausgewählt ist.

12. Zusammensetzung nach Anspruch 11, **dadurch gekennzeichnet, daß** die ergänzende Oxidationsbase(n) 0,0005 bis 12 Gew.-% des Gesamtgewichts der Farbmittelzusammensetzung ausmachen.

13. Zusammensetzung nach einem der Ansprüche 8 bis 12, **dadurch gekennzeichnet, daß** sie mindestens einen Kuppler und/oder mindestens einen Direktfarbstoff enthält.

14. Zusammensetzung nach Anspruch 13, **dadurch gekennzeichnet, daß** der oder die Kuppler unter den m-Phenylendiaminen, m-Aminophenolen, m-Dihydroxybenzolen und heterocyclischen Kupplern und deren Additionssalzen mit einer Säure ausgewählt sind.

15. Zusammensetzung nach Anspruch 14, **dadurch gekennzeichnet, daß** der oder die Kuppler unter 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis-(2,4-diaminophenoxy)-propan, Sesamol, α-Naphthol, 6-Hydroxyindol, 4-Hydroxyindol, 4-Hydroxy-N-methylindol, 6-Hydroxyindolin, 2,6-Dihydroxy-4-methylpyridin, 1-H-3-Methyl-pyrazol-5-on, 1-Phenyl-3-methyl-pyrazol-5-on und deren Additionssalzen mit einer Säure ausgewählt sind.

16. Zusammensetzung nach einem der Ansprüche 13 bis 15, **dadurch gekennzeichnet, daß** der oder die Kuppler etwa 0,0001 bis 10 Gew.-% des Gesamtgewichts der Farbmittelzusammensetzung ausmachen.

17. Zusammensetzung nach einem der Ansprüche 8 bis 17, **dadurch gekennzeichnet, daß** die Additionssalze mit einer Säure unter den Hydrochloriden, Hydrobromiden, Sulfaten, Citraten, Succinaten, Tartraten, Lactaten und Acetaten ausgewählt sind.

18. Verfahren zum Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 8 bis 17 aufgebracht wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgebracht wird.

19. Verfahren nach Anspruch 18, **dadurch gekennzeichnet, daß** das Oxidationsmittel unter Wasserstoffperoxid, Harnstoffperoxid, Alkalimetallbromaten, Salzen von Persäuren und Enzymen ausgewählt ist.

20. Vorrichtung mit mehreren Abteilungen oder "Kit" zum Färben mit mehreren Abteilungen, wobei eine erste Abteilung eine Farbmittelzusammensetzung nach einem der Ansprüche 8 bis 17 und eine zweite Abteilung eine oxidierende Zusammensetzung enthält.
